# EUROPÄISCHE PATENTANMELDUNG

(11) **EP 3 260 870 A1**
(43) Veröffentlichungstag der Anmeldung: **27.12.2017**
(21) Anmeldenummer: 17176704.9
(22) Anmeldetag: 19.06.2017
(51) Int. Cl.: G01N 35/10, G01N 15/10

(54) **VORRICHTUNG UND VERFAHREN ZUM DETEKTIEREN VON ZELLEN ODER PARTIKELN IN EINEM FLUIDBEHÄLTER**

(30) Priorität: 21.06.2016 DE 102016211038
(71) Anmelder: CYTENA GMBH, 79110 Freiburg (DE)
(72) Erfinder: SCHÖNDUBE, Jonas, 79098 Freiburg (DE); GROSS, Andre, 79104 Freiburg (DE)
(74) Vertreter: Stöckeler, Ferdinand

(57) **Zusammenfassung**

Eine Vorrichtung zum Detektieren von Zellen oder Partikeln in einem Fluidbehälter weist einen Dispenser auf, der ausgelegt ist, um zumindest eine Zelle oder zumindest einen Partikel in ein definiertes Teilvolumen eines Fluids abzugeben, mit dem der Fluidbehälter zumindest teilweise gefüllt ist, und eine Detektionsvorrichtung, die ausgelegt ist, um zeitlich koordiniert mit der Abgabe der zumindest einen Zelle oder des zumindest einen Partikels durch den Dispenser eine Detektion in dem definierten Teilvolumen und/oder in einem oder mehreren Teilvolumen unterhalb des definierten Teilvolumens durchzuführen, um die zumindest eine Zelle oder den zumindest einen Partikel beim Eintritt in das Fluid oder unmittelbar nach dem Eintritt in das Fluid zu erfassen.

## Beschreibung

Die vorliegende Erfindung betrifft Vorrichtungen und Verfahren zum Detektieren von Zellen oder Partikeln, die von einem Dispenser in ein definiertes Teilvolumen eines in einem Fluidbehälter befindlichen Fluids abgegeben werden.

Generell ist es häufig erforderlich, nach dem Einbringen von Zellen oder Partikeln in einen Fluidbehälter zu erfassen, ob sich die Zelle oder der Partikel tatsächlich in dem Fluidbehälter befindet. Beispielsweise werden monoklonale Antikörper und andere Proteine, die nachfolgend Produkte genannt werden, mithilfe sogenannter monoklonaler Zelllinien hergestellt. Dies sind Populationen aus Zellen, die von einer einzelnen Zelle abstammen. So kann möglichst gut sichergestellt werden, dass alle Zellen der Population einen annähernd gleichen Genotypen vorweisen und damit ein möglichst gleiches Produkt erzeugen.

Um eine monoklonale Zeillinie zu erzeugen, werden Zellen einzeln in sogenannte Mikrotiterplatten überführt und vermehren sich dort kontrolliert, bis die gewünschte Populationsgröße erreicht ist. Das Ablegen einzelner Zellen in die Mikrotiterplatten geschieht durch Freistrahldruckmethoden oder Pipettieren einzelner Zellen in die einzelnen Näpfchen oder Kavitäten der Mikrotiterplatte, die im Folgenden als "Wells" bezeichnet werden. Diese Wells stellen Behälter dar. Bei der Produktion therapeutischer Produkte aus Zellkulturen muss aus regulatorischen Gründen nachgewiesen werden, dass sich zu Beginn des Prozesses wirklich nur eine Zelle im Well befunden hat. Dabei ist es wichtig, dass der Wellboden ausreichend groß, d. h. bedeutend größer als eine Zelle ist, um das Wachsen der Population bis hin zur benötigten Größe zu erlauben. Schlussendlich wird aus einer Serie von einigen Hunderten bis Tausenden solcher Klonpopulationen derjenige in die Produktion überführt, der das gewünschte Produkt am stabilsten und in größter Menge herstellt.

Verfahren zum Erfassen von Zellen in Fluidbehältern, beispielsweise den Wells einer Mikrotiterplatte, sind aus dem Stand der Technik bekannt.

K. Evans et al. beschreiben in "Assurance of monoclonality in one round of cloning through cell sorting for single cell deposition coupled with high resolution cell imaging", 2015, American Institute of Chemical Engineers, Biotechnol. Prog., vol. 00, No. 00, http://doi.org/10.1002/btpr.2145, einen Prozess zur Herstellung monoklonaler Zelllinien. Zellen werden mithilfe eines sogenannten FACS-Geräts (FACS = fluorescent activated cell sorting = Fluoreszenz-aktivierte Zellsortierung) in das Well einer Mikrotiterplatte überführt. Diese wird danach zentrifugiert, um die Zellen auf dem Boden zu befördern. Anschließend wird der gesamte Wellboden mikroskopiert, typischerweise mittels eines sogenannten Imagers, und einzelne Zellen werden darin gesucht, was durch den Benutzer geschieht. Dabei ist es äußerst schwierig, eine einzelne Zelle in dem großen Observationsvolumen zu erkennen.

Die Durchflusszytometrie stellt ein bekanntes Verfahren zur Analyse von Zellen dar, die eine elektrische Spannung oder einen Lichtstrahl passieren. Beispielsweise ist in der US 3,380,584 A ein Verfahren zur Partikeltrennung beschrieben, bei dem ein Druckverfahren mit kontinuierlichem Strahl (Continous Jet) angewendet wird, was den Nachteil hat, dass kontinuierlich Tropfen erzeugt werden, ohne den Tropfenstrom kontrolliert unterbrechen zu können. Bei der gezielten Sortierung von Zellen oder Partikeln mittels dieser Technik ist es folglich notwendig, die Tropfen je nach Inhalt an verschiedenen Positionen abzulegen. Dies geschieht durch eine elektrostatische Ablenkung im Flug. Je höher dabei die Anzahl der Positionen und die geforderte Ablagegenauigkeit (beispielsweise in 96- oder 384-Well-Platten), desto schwieriger und technischer aufwändiger ist der Prozess. Aus der EP 0 421 406 A2 sind Vorrichtungen und Verfahren zur Partikeltrennung bekannt, bei denen ein thermischer Druckkopf verwendet wird, um Partikel zu dispensieren. Die Partikel sind willkürlich im Reservoir angeordnet und werden nach dem Ausstoßen im Flug optisch analysiert. Die oben beschriebenen Verfahren erlauben es, Zellen einzeln abzulegen, können dabei aber keine 100-prozentige Effizienz erreichen. Deshalb müssen die Mikrotiterplatten im Nachhinein mit sogenannten Imagern mikroskopiert werden.

Vorrichtungen und Verfahren zur Erfassung von Zellen und Partikeln in Mikrotiterplatten sind aus der US 7,310,147 B2, der EP 1 686 368 A2, der US 8,417,011 B2 und der US 8,795,981 B2 bekannt.

Die US 7,646,482 B2 beschreibt ein Verfahren zum automatischen Auffinden der richtigen Fokusebene, um beispielsweise Zellen an einem Wellboden zu mikroskopieren. Das Verfahren detektiert dabei Muster im Sensorsignal, während das Mikroskop durch den Boden der Platte fokussiert.

Die US 8,383,042 B2 beschreibt einen Imager (Bilderzeugungsgerät) mit einem Vakuumhalter. Der Vakuumhalter saugt die Mikrotiterplatte an, um den Boden der Mikrotiterplatte flach zu halten, und sorgt so für eine geringere Varianz des Abstands des Wellbodens zum Objektiv.

Vorrichtungen und Verfahren zum Abgeben einer Zelle bzw. eines Partikels in einem freifliegenden Tröpfchen sind aus der WO 2011/154042 A1 bekannt.

Die Erfinder haben erkannt, dass gegenwärtige Dispensiermethoden zur Einzel-Ablage Zellen mit hoher Effizienz detektieren und ablegen können, wobei sie jedoch nicht zu 100 % zuverlässig sind.

Die Aufgabe der vorliegenden Erfindung besteht darin, Vorrichtungen und Verfahren zu schaffen, die Zellen oder Partikel in einem Fluidbehälter mit hoher Zuverlässigkeit detektieren können.

Diese Aufgabe wird durch eine Vorrichtung gemäß Anspruch 1 und ein Verfahren gemäß Anspruch 11 gelöst.

Ausführungsbeispiele der Erfindung schaffen eine Vorrichtung zum Detektieren von Zellen oder Partikeln in einem Fluidbehälter, mit folgenden Merkmalen:
einem Dispenser, der ausgelegt ist, um zumindest eine Zelle oder zumindest einen Partikel in ein definiertes Teilvolumen eines Fluids abzugeben, mit dem der Fluidbehälter zumindest teilweise gefüllt ist;
einer Detektionsvorrichtung, die ausgelegt ist, um zeitlich koordiniert mit der Abgabe der zumindest einen Zelle oder des zumindest einen Partikels durch den Dispenser eine Detektion in dem definierten Teilvolumen und/oder in einem oder mehreren Teilvolumen unterhalb des definierten Teilvolumens durchzuführen, um die zumindest eine Zelle oder den zumindest einen Partikel beim Eintritt in das Fluid oder unmittelbar nach dem Eintritt in das Fluid zu erfassen.

Ausführungsbeispiele der Erfindung schaffen ein Verfahren zum Detektieren von Zellen oder Partikeln in einem Fluidbehälter, mit folgenden Merkmalen:
Abgeben zumindest einer Zelle oder zumindest eines Partikels in ein definiertes Teilvolumen eines Fluids, mit dem ein Fluidbehälter zumindest teilweise gefüllt ist; und
Durchführen einer Detektion in dem definierten Teilvolumen und/oder in einem oder mehreren Teilvolumen unterhalb des definierten Teilvolumens zeitlich koordiniert mit der Abgabe der zumindest einen Zelle oder des zumindest einen Partikels, um die zumindest eine Zelle oder den zumindest einen Partikel beim Eintritt in das Fluid oder unmittelbar nach dem Eintritt in das Fluid zu erfassen.

Bei Ausführungsbeispielen der Erfindung wird das Objekt, also die Zelle oder der Partikel, beispielsweise die einzelne Zelle oder der einzelne Partikel nicht innerhalb des Dispensers und nicht in einem freiliegenden Tropfen, in dem das Objekt abgegeben wird, detektiert. Bei Ausführungsbeispielen findet somit die Detektion nicht während des Transport, z.B. in das Well einer Mikrotiterplatte, statt. Vielmehr wird die Zelle oder der Partikel beim Eintritt in das Fluid oder unmittelbar nach dem Eintritt in das Fluid, das den Fluidbehälter, in dem die Zelle oder Partikel erfasst werden soll, zumindest teilweise füllt, erfasst. Somit ermöglichen Ausführungsbeispiele einen zuverlässigen Nachweis, dass die Zelle oder der Partikel in den Fluidbehälter gelangt ist und sich tatsächlich in dem Fluidbehälter befindet. Bei Ausführungsbeispielen gibt der Dispenser einen freifliegenden Tropfen ab, in dem das Objekt, also die Zelle oder der Partikel, verkapselt ist, so dass bei solchen Ausführungsbeispielen ein zuverlässiger Nachweis, dass der Tropfen tatsächlich in dem Fluidbehälter, beispielsweise einem Well einer Mikrotiterplatte, gelandet ist, möglich ist.

Ausführungsbeispiele der Erfindung werden nachfolgend Bezug nehmend auf die beiliegenden Zeichnungen näher erläutert. Es zeigen:
- Fig. 1: eine schematische Darstellung einer Vorrichtung zum Detektieren von Zellen oder Partikeln in einem Fluidbehälter;
- Fig. 2: eine schematische Darstellung einer Vorrichtung mit einem optischen Sensor;
- Fig. 3: eine schematische Darstellung eines Fluidbehälters;
- Fig. 4: ein Flussdiagramm eines Ausführungsbeispiels eines Verfahrens zum Detektieren von Zellen oder Partikeln in einem Fluidbehälter;
- Fig. 5: eine Gegenüberstellung eines Ausführungsbeispiels eines hierin beschriebenen Verfahrens zum Detektieren von Zellen oder Partikeln in einem Fluidbehälter mit einem bekannten Verfahren; und
- Fig. 6: schematische Darstellungen von Fluidbehältern zur Erläuterung von bei bekannten Verfahren auftretenden Problemen.

Bevor nachfolgend auf Ausführungsbeispiele eingegangen wird, sei zunächst angemerkt, dass die Erfindung durch diese speziellen Ausführungsbeispiele nicht beschränkt ist, sondern durch den Wortlaut der Ansprüche.

Ferner werden zunächst einige hierin verwendete Begriffe erklärt. Unter einem Dispenser wird eine Apparatur verstanden, die zum Abgeben von Zellen oder Partikeln ausgelegt ist. Bei Beispielen von Dispensern kann es sich um Tropfengeneratoren handeln, die zum Abgeben von Flüssigkeitsmengen in Form von frei fliegenden Tropfen ausgelegt sind. Unter einer Drop-on-Demand-Drucktechnologie wird eine Drucktechnologie verstanden, die das gezielte Erzeugen einzelner Tropfen aus einer Düse zu einem gewählten Zeitpunkt ermöglicht. Im Gegensatz dazu wird unter einer Continous-Jet-Drucktechnologie eine Drucktechnik verstanden, bei der druckgetrieben ein dünner, kontinuierlicher Flüssigkeitsstrahl aus einer Düse abgegeben wird. Durch Anlegen einer hochfrequenten Schwingung an die Düse zerfällt der Strahl nach Austritt in einzelne Tropfen, die unter anderem elektrostatisch abgelenkt werden können. Unter einem Observationsvolumen wird hierin ein Volumenbereich bestimmter Höhe verstanden, in dem Messungen oder Beobachtungen vorgenommen werden. Observationsvolumen können in einem definierten, zweidimensionalen Raster bestimmter Höhe angeordnet sein. Unter einer Mikrotiterplatte wird eine Platte verstanden, die viele voneinander isolierte Kavitäten (Wells) in Reihen und Spalten enthält. Mikrotiterplatten sind häufig rechteckig und bestehen in der Regel aus Kunststoff. Unter einem Imager wird ein Bilderzeugungsgerät verstanden, beispielsweise ein automatisiertes Mikroskop, das beispielsweise das Mikroskopieren ganzer Mikrotiterplatten ermöglicht. Dabei sind Imager ausgelegt, um jedes Well der Mikrotiterplatte einzeln in hoher Auflösung zu fotografieren. Dies ermöglicht dem Benutzer anschließend, einzelne Zellen in den Wells aufzuspüren.

Wie oben ausgeführt wurde, bleiben bekannte Systeme den Nachweis, dass ein abgegebener Tropfen mit einer Zelle bzw. einem Partikel auch tatsächlich in dem Well einer Mikrotiterplatte gelandet ist, schuldig. Hierfür wurde in der Regel immer eine Sekundärtechnologie, wie beispielsweise ein Imager eingesetzt. Demnach weiß der Benutzer später nicht, ob die gewünschte Einzelzelle tatsächlich das Well erreicht hat, solange er nur das Dispensiersystem einsetzt.

Ferner wurde erkannt, dass beim Einsatz von Imagern weitere Probleme auftreten. So ist die vom Imager zu fotografierende Fläche im Vergleich zu einer Zelle riesig, mit einem Faktor von ca. 1:1.000.000. Um sicherzustellen, dass nur eine Zelle im Well ist, muss zwingend die gesamte Wellfläche gescannt werden. Gleichzeitig muss die Auflösung dabei so hoch sein, dass man die Zelle sicher als solche identifizieren kann. Je höher die Auflösung, desto kleiner das Bildfeld, desto länger dauert der Scan. Ferner erfordert das Scannen, dass mehrere Bilder örtlich versetzt zueinander aufgenommen werden und diese dann zu einem Gesamtbild zusammengesetzt werden. Liegt eine Zelle nun genau zwischen zwei solchen Bildern, kann es vorkommen, dass die Darstellung der Zelle durch das Zusammensetzen abgeschnitten wird oder schlimmstenfalls sogar verschwindet. Ferner ist aufgrund der hohen optischen Auflösung die optische Fokustiefe limitiert. Ist das System nicht genau fokussiert, verschwimmen Zellen und können im schlimmsten Fall übersehen werden. Der Scan des gesamten Wellvolumens, d.h. aller Fokusebenen vom Boden bis zu Oberfläche der Flüssigkeit, ist zeitlich und datentechnisch sehr aufwändig und somit kaum umsetzbar. Es würde mit gegenwärtiger Imager-Technik ca. zehn Stunden dauern, eine 96-Wellplatte zu scannen. Das entstehende Datenvolumen betrüge ca. 40 GB. Hochgerechnet auf die Menge der Platten und die Zeit, welche die Daten gespeichert bleiben müssten, wäre dies nicht zu bewerkstelligen. Ferner ist es notwendig, die Zellen vor dem Scan in die Fokusebene, d. h. auf den Wellboden zu befördern. Dies wird typischerweise durch Zentrifugieren nach der Zellablage erreicht. Hierzu werden die Platten bei hohen Fliehkräften geschleudert, bis die Zellen sich am Boden befinden. Durch Auftreten radialer Fliehkräfte beim Beschleunigen und Abbremsen können Zellen nach außen transportiert werden. Sie kommen dann häufig in den Ecken der Wells zu liegen. Dort sind sie sehr schwer zu identifizieren. Mikrotiterplatten sind trotz weitest gehender Normung mit Fertigungstoleranzen behaftet. Die Höhe des Wellbodens kann sowohl insgesamt als auch von Well zu Well schwanken. Dadurch sind die Ebene, in der die Zellen liegen und damit der Fokus unter Umständen nicht einheitlich. Durch einen falschen Fokus können die Objekte zum Rand hin verschmieren.

Durch die Art der Fertigung der Mikrotiterplatten durch Spritzguss oder Tiefziehen haben die Wells in der Regel Entformschrägen und die Ecken am Boden sind nicht 90°, sondern immer leicht rund. Dies führt im Imager zu starken Beugungs- und Abschattungseffekten.

Somit sind Zellen, die in den Ecken zu liegen kommen, ggf. nicht eindeutig oder gar nicht identifizierbar. Wird der Wellboden zum Rand hin höher, sind Zellen nur mittig im Fokus und außen unscharf. Ferner können durch Brechungseffekte einzelne Zellen zweimal abgebildet werden. Es kann ein sogenanntes Geisterbild der Zelle in direkter Nachbarschaft des eigentlichen Bildes entstehen. Somit kann aber nicht mit Sicherheit ausgesagt werden, ob es sich um ein solches Geisterbild oder tatsächlich um eine zweite Zelle handelt.

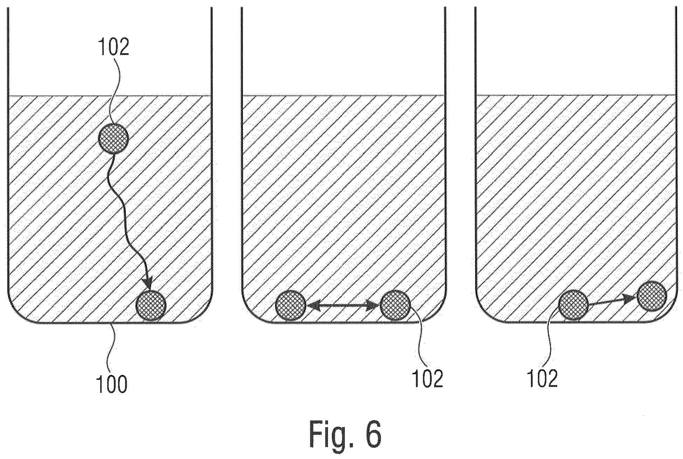

Wie oben ausgeführt wurde, werden die Mikrotiterplatten in der Regel von einem Dispensiersystem zu einem Imager-System verbracht, um dort zu überprüfen, ob Zellen oder Partikel in den Wells der Mikrotiterplatten angeordnet sind. Durch die notwendigen Schritte des Zentrifugierens und des Wechselns der Mikrotiterplatten zwischen Geräten, sowie durch die vergehende Zeit zwischen Ablage der Zelle durch Dispensieren und dem Erfassen in dem Well können die Zellen im Well praktisch überall am Boden des Well landen. Fig. 6 zeigt im linken Bereich einen Fluidbehälter 100, in dem eine Zelle 102 durch eine Sedimentation aufgrund einer verstrichenen Zeit auf den Boden des Fluidbehälters 100, beispielsweise des Well der Mikrotiterplatte, abgesunken ist. Die mittlere Darstellung in Fig. 6 zeigt eine Bewegung der Zelle 102 auf dem Boden durch einen Transport. Die rechte Darstellung in Fig. 6 zeigt eine Bewegung der Zelle 102 durch Fliehkräfte, wie sie beispielsweise durch Zentrifugieren auftreten können. Diese Ausführungen zeigen, dass die Zellen in dem Well praktisch überall am Boden landen können, wobei sie häufig tatsächlich in den Ecken landen.

Ein Ausweichen auf andere Substrate mit kleinerer Wellbodenfläche würde einige Probleme, beispielsweise das Zusammensetzen der Bilder, theoretisch beheben. Jedoch hat dies andere Nachteile. Das Verhältnis zwischen ebener Fläche des Wellbodens zu unebenem Randbereich, in dem Abschattung und Defokussierung auftreten, würde sich ins Negative verschieben. Die Wahrscheinlichkeit, dass eine Zelle am Rand zu liegen kommt, würde ansteigen. Die Fläche am Wellboden bzw. vor allem das Volumen des Well, welches den Zellen zum Wachstum zur Verfügung steht, wäre deutlich kleiner. Zellen würden schlechter wachsen und könnten keine ausreichend großen Populationen bilden. Dies würde den gesamten Arbeitsablauf aufwändiger machen. Aufgrund des geringen Wellvolumens müsste Medium getauscht bzw. aufgefüllt werden, um die Zellen ausreichend lange mit Nährstoffen zu versorgen. Die Gefahr von Querkontamination oder die Wahrscheinlichkeit eines frühzeitiges Absterbens der Population würden steigen.

Die Erfinder haben somit erkannt, dass eine Dispensiertechnologie alleine nicht ausreichend für den Nachweis einer Einzelzelle in einem Fluidbehälter, beispielsweise einem Well einer Mikrotiterplatte, ist. Ferner sind Imager aufgrund der oben beschriebenen Problematiken ebenfalls in ihrer Aussagekraft diesbezüglich limitiert. Letzten Endes kann dies dazu führen, dass die für die Produktion ausgewählte, optimale Population nicht sicher als monoklonal nachgewiesen werden kann und somit verworfen werden muss. Dies führt überdies zu hohen wirtschaftlichen Risiken und hohen Kosten.

Ausführungsbeispiele der Erfindung schaffen Vorrichtungen und Verfahren, die die oben beschriebenen Probleme beheben und die eine zuverlässige Detektion auch von einzelnen Zellen oder Partikeln in einem Fluidbehälter ermöglichen.

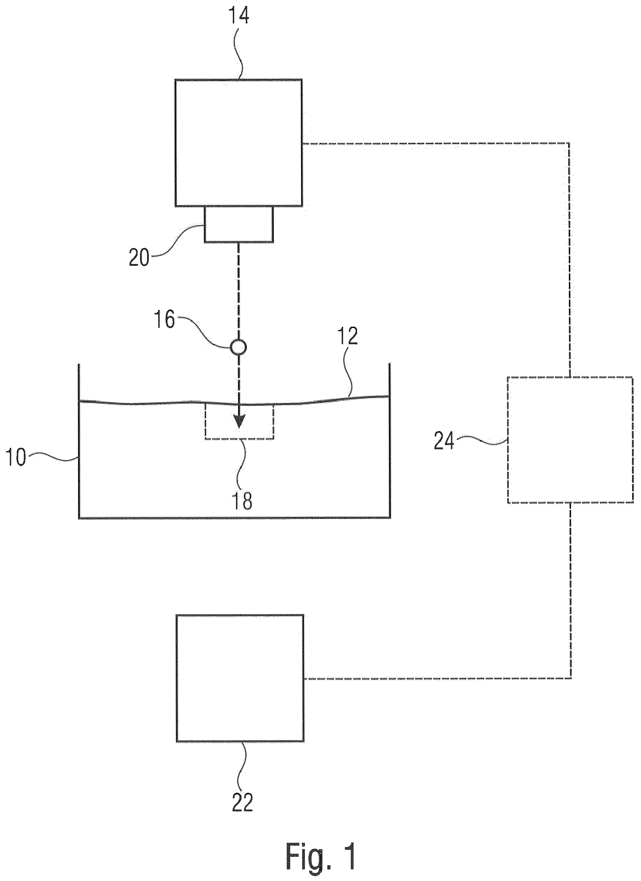

Ein Ausführungsbeispiel einer Vorrichtung zum Detektieren von Zellen oder Partikeln ist in Fig. 1 gezeigt. Die Vorrichtung umfasst einen Fluidbehälter 10. Bei dem Fluidbehälter kann es sich beispielsweise um einen Well (Kavität) einer Mikrotiterplatte handeln, die ein Array entsprechender Wells aufweist. Der Fluidbehälter kann einen Boden und Seitenwände, die ein Volumen des Fluidbehälters begrenzen und ermöglichen, dass der Fluidbehälter mit einem Fluid zumindest teilweise füllbar ist, aufweisen. Bei Ausführungsbeispielen besteht der Fluidbehälter 10 aus einem transparenten Material. Der Fluidbehälter 10 ist zumindest teilweise mit einem Fluid 12, beispielsweise einer Flüssigkeit gefüllt. Bei der Flüssigkeit kann es sich beispielsweise um eine Nährlösung für eine Zellkultur handeln. Im Folgenden wird allgemein von einer Flüssigkeit 12 gesprochen. Die Vorrichtung weist ferner einen Dispenser 14 auf, der ausgelegt ist, um zumindest eine Zelle oder Partikel 16 in ein definiertes Teilvolumen 18 der Flüssigkeit 12 abzugeben. Bei dem Dispenser 14 kann es sich um einen Drop-on-Demand-Dispenser handeln, der konfiguriert ist, um einen einzelnen Tropfen, in dem eine Zelle bzw. ein Partikel eingekapselt ist, aus einer Düse 22 abzugeben. Der Dispenser 14 ist so relativ zu dem Fluidbehälter 10 positioniert bzw. positionierbar, dass die Zelle bzw. der Partikel in das definierte Teilvolumen 18 abgegeben wird. Der Dispenser kann ausgelegt sein, um entsprechend einzelne Tropfen aus einer Zellsuspension oder Partikelsuspension abzugeben, und kann einen Aufbau aufweisen, wie er beispielsweise in der WO 2011/154042 A1 beschrieben ist.

Die Vorrichtung weist ferner eine Detektionsvorrichtung 22 auf, die ausgelegt ist, um zeitlich koordiniert mit der Abgabe der zumindest einen Zelle 16 (oder des zumindest einen Partikels) durch den Dispenser 14 eine Detektion in dem definierten Teilvolumen 18 und/oder in einem oder mehreren Teilvolumen unterhalb des definierten Teilvolumens durchzuführen, um die zumindest eine Zelle 16 beim Eintritt in die Flüssigkeit 12 oder unmittelbar nach dem Eintritt in die Flüssigkeit 12 zu erfassen.

Bei Beispielen ist der Fluidbehälter Teil der Vorrichtung. Bei Beispielen ist der Fluidbehälter nicht Teil der Vorrichtung und kann separat von der Vorrichtung bereitgestellt werden.

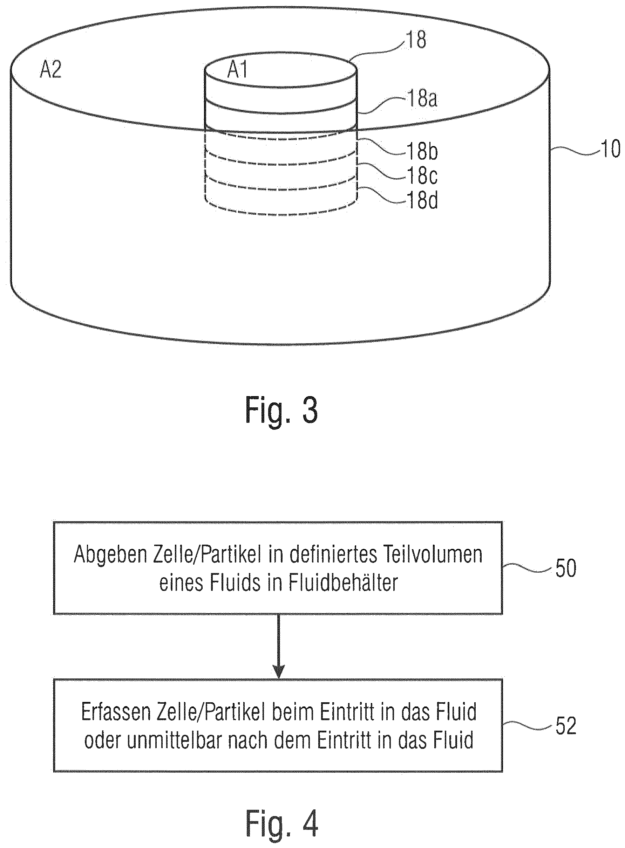

Hinsichtlich der Erläuterung des definierten Teilvolumens und des einen oder mehreren unterhalb des definierten Teilvolumens angeordneten Teilvolumen wird auf Fig. 3 verwiesen, die eine schematische perspektivische Ansicht des Fluidbehälters 10 zeigt. Die Teilvolumen sind in Fig. 3 als Scheiben dargestellt, wobei das definierte Teilvolumen 18 durch die oberste Scheibe gebildet ist. Das definierte Teilvolumen 18 kann sich beispielsweise von der Flüssigkeitsoberfläche der in dem Fluidbehälter 10 angeordneten Flüssigkeit um eine vorbestimmte Tiefe nach unten erstrecken. Die Tiefe jedes Teilvolumens kann dabei der Schärfentiefe einer Fokusebene der Detektionsvorrichtung entsprechen. Diese kann beispielsweise in einer Größenordnung von 40 µm bis 60 µm liegen. Bei der Darstellung in Fig. 3 wird aus Vereinfachungsgründen angenommen, dass die Flüssigkeit den Behälter 10 vollständig füllt. In der Realität wird der Behälter in der Regel nur teilweise mit der Flüssigkeit gefüllt sein. Wie in Fig. 3 zu sehen ist, ist die Fläche A1 des definierten Teilvolumens 18 deutlich geringer als die Fläche A2 des Fluidbehälters 10. Unter Fläche wird dabei jeweils die Fläche parallel zu der Flüssigkeitsoberfläche verstanden. Die ein oder mehreren Teilvolumen unterhalb des definierten Teilvolumens 18 sind unterhalb des Teilvolumens 18 in zunehmender Tiefe angeordnet. Die Teilvolumen können in einer Richtung, die senkrecht zu der Flüssigkeitsoberfläche ist, vollständig überlappend sein. Die Teilvolumen 18 und 18a - 18d können jeweils so ausgebildet sein, dass eine Detektion durch die Detektionsvorrichtung 22 in dem gesamten Teilvolumen stattfinden kann. Beispielsweise sind die Teilvolumen so dimensioniert, dass ein bildgebender Sensor ein fokussiertes Bild des jeweiligen Teilvolumens erzeugen kann.

Der Dispenser 14 und die Detektionsvorrichtung 22 können mit einer Steuerung 24 verbunden sein, die die Abgabe der Zelle bzw. des Partikels durch den Dispenser und die Detektion durch die Detektionsvorrichtung 22 zeitlich koordiniert. Bei Ausführungsbeispielen kann die Steuerung in dem Dispenser oder der Detektionsvorrichtung angeordnet sein. Die Steuerung kann, wie für Fachleute offensichtlich ist, beispielsweise durch eine entsprechend programmierte Recheneinrichtung oder eine anwenderspezifische integrierte Schaltung implementiert sein. Bei Ausführungsbeispielen kann die Detektionsvorrichtung ausgelegt sein, um die Erfassung der zumindest einen Zelle oder des zumindest einen Partikels spätestens zehn Sekunden nach dem Eintritt in das Fluid, bei dem gezeigten Ausführungsbeispiel die Flüssigkeit 12, zu erfassen. Beispielsweise kann die Detektionsvorrichtung ausgelegt sein, um eine Detektion in einem der Teilvolumen dann durchzuführen, wenn erwartet wird, dass sich die Zelle bzw. der Partikel in dem Teilvolumen befindet. Falls die Detektion in dem direkt unter der Oberfläche der Flüssigkeit 12 angeordneten Teilvolumen 18 erfolgt, kann die Erfassung unmittelbar nach der Abgabe von dem Dispenser erfolgen, beispielsweise innerhalb einer Sekunde oder einem noch geringeren Zeitraum. Bei Ausführungsbeispielen der Erfindung ist die Detektionsvorrichtung ausgelegt, um die Zelle bzw. den Partikel in einem Teilvolumen zu erfassen, das oberhalb des Bodens des Fluidbehälters angeordnet ist. Somit kann das Objekt erfasst werden, bevor es den Boden des Fluidbehälters erreicht hat.

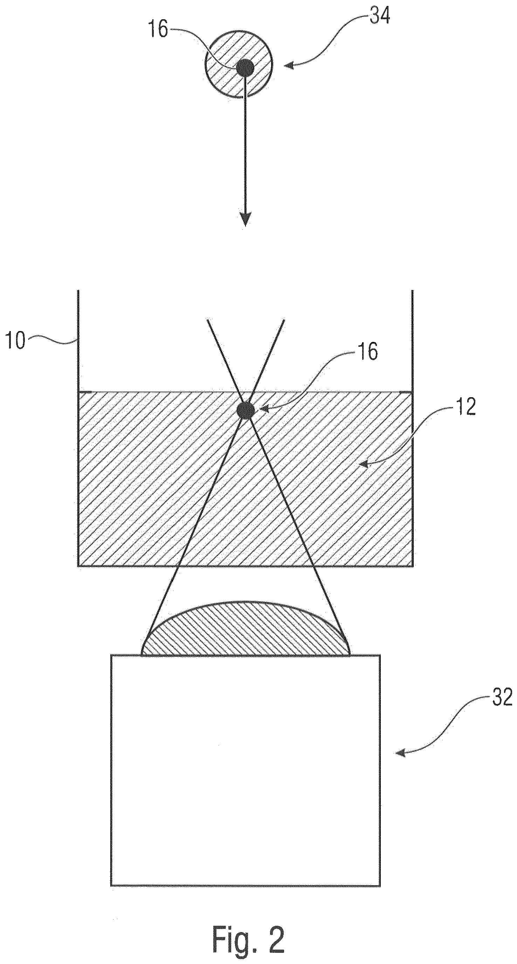

Fig. 2 zeigt ein Ausführungsbeispiel einer Vorrichtung zum Detektieren von Zellen oder Partikeln, bei der die Detektionsvorrichtung einen optischen Sensor 32 aufweist. Ein Dispenser (in Fig. 2 nicht gezeigt) ist zum Abgeben von Tropfen 34 einer Zellsuspension ausgelegt, wobei in dem Tropfen 34 eine Zelle 16 angeordnet ist. Der Dispenser gibt den Tropfen in den Fluidbehälter 10, beispielsweise in der Form eines Wells einer Mikrotiterplatte, das mit einem Zellmedium 12 vorbefüllt ist, ab. Bei Ausführungsbeispielen kann die Form des Fluidbehälters so gewählt sein, dass die Oberfläche der Flüssigkeit flach ist. Unter dem transparenten Fluidbehälter 10 befindet sich ein optischer Sensor 32, beispielsweise ein Mikroskop mit verstellbarem Fokus. Eine Zelle/ein Partikel wird bereits beim Eintritt in die Flüssigkeit in dem Fluidbehälter 10 mit dem optischen Sensor aufgenommen. Der optische Sensor weist eine Kamera und eine Optik auf. Der optische Sensor 32 fokussiert das System durch den transparenten Fluidbehälter hindurch. Dazu fokussiert der optische Sensor 32, der eine Bildaufnahmevorrichtung darstellt, auf ein definiertes Teilvolumen direkt unterhalb der Oberfläche der Flüssigkeit 12. Beispielsweise kann der optische Sensor 32 auf ein Teilvolumen direkt unterhalb der Flüssigkeitsoberfläche oder auf ein Teilvolumen in einer Tiefe von weniger als 5 mm von der Oberfläche der Flüssigkeit 12 fokussieren. Ziel ist es dabei, zu verifizieren, dass die Zelle/der Partikel tatsächlich im Reservoir landet. Es muss nicht gewartet werden, bis die Zelle/der Partikel zum Boden des Reservoirs abgesunken ist. Wohin die Zelle/der Partikel letzten Endes in dem Fluidbehälter, auf dem Boden desselben, zu liegen kommt, spielt bei der beschriebenen Erfassung keine Rolle.

Bei Ausführungsbeispielen können einzelne Tropfen einer Zell- oder Partikelsuspension, die jeweils ein Volumen von ca. 200 pl aufweisen können, in eine Well einer Mikrotiterplatte abgegeben werden. Das Well kann vorher beispielsweise hälftig mit einer Flüssigkeit befüllt werden, wobei der Fokus des optischen Sensors konstant unterhalb der Flüssigkeitsoberfläche gehalten werden kann. So kann die Zelle/der Partikel durch die Fokusebene des optischen Sensors 32, bei dem es sich um ein Lichtmikroskop handeln kann, sedimentieren. Das Aufnehmen eines Bildes zeitlich koordiniert mit der Abgabe der Zelle/des Partikels erlaubt somit die sichere Detektion der Zelle/des Partikels in dem Fluidbehälter. Ferner kann bei Ausführungsbeispielen eine Bilderserie des definierten Teilvolumens (bzw. mehrerer definierten Teilvolumen) aufgenommen werden, um eine noch sicherere Detektion der Zelle/des Partikels zu ermöglichen. Praktische Versuche haben gezeigt, dass es unter Verwendung eines solchen Aufbaus ohne weiteres möglich ist, zu erkennen, dass das Objekt (die Zelle/der Partikel) im Observationsvolumen (dem definierten Teilvolumen) in die Flüssigkeit eindringt und dann Richtung Boden sedimentiert, wobei es von dem optischen Sensor beobachtet werden kann.

Bei alternativen Ausführungsbeispielen kann die Fokusebene des optischen Sensors auch entgegen der Sedimentationsbewegung des Objekts bewegt werden, um so die Zeit bis zur Detektion zu verringern. Anders ausgedrückt kann die Detektionsvorrichtung ausgelegt sein, um beginnend mit einem Teilvolumen in einer größeren Tiefe nacheinander Detektionen in mehreren Teilvolumen mit jeweils abnehmender Tiefe durchzuführen.

Das Durchführen mehrerer Detektionen, beispielsweise durch eine Bildaufnahmevorrichtung, ermöglicht, dass das Objekt bei der Durchführung zumindest einer Detektion genau im Fokus ist und somit absolut zuverlässig detektiert werden kann. Praktische Versuche haben ergeben, dass beispielsweise ein Polystyrolpartikel mit einem Durchmesser von 15 µm durch eine entsprechende Vorgehensweise ohne weiteres erfasst werden konnte.

Mehrere Teilvolumen jeweils abnehmender Tiefe sind beispielsweise in Fig. 3 der vorliegenden Anmeldung gezeigt und mit dem Bezugszeichen 18a - 18d versehen. Beispielsweise könnte unmittelbar nach der Abgabe des Tropfens durch den Dispenser mit einer Detektion in dem Teilvolumen 18d begonnen werden und dann Detektionen in jeweiligen Teilvolumen mit abnehmender Tiefe, d. h. von 18c zu 18b zu 18a zu 18. Dadurch ist es möglich, eine Zelle oder einen Partikel, der in die Flüssigkeit 12 in dem Fluidbehälter 10 eingebracht wurde, mit hoher Zuverlässigkeit zu erfassen.

Bei alternativen Ausführungsbeispielen könnte die Detektionsvorrichtung auch ausgelegt sein, um beginnend mit einem Teilvolumen in einer geringeren Tiefe nacheinander Detektionen in mehreren Teilvolumen mit jeweils zunehmender Tiefe durchzuführen.

Bei Ausführungsbeispielen der Erfindung kann das definierte Teilvolumen eine Fläche von weniger als 10 mm² aufweisen. Beispielsweise kann das Teilvolumen eine Fläche von 2x2 mm bei einer Tiefe, die der Tiefenschärfe einer Fokusebene der Detektionsvorrichtung entspricht. Bei Ausführungsbeispielen kann die Detektionsvorrichtung ausgebildet sein, um eine Bildsequenz in einem Teilvolumen aufzunehmen, bis die Zelle oder der Partikel in das Teilvolumen sedimentiert. Bei Ausführungsbeispielen kann die Detektionsvorrichtung ausgebildet sein, um auf ein Teilvolumen, das direkt unterhalb der Flüssigkeitsoberfläche angeordnet ist, oder auf ein Teilvolumen, das in einer Tiefe von weniger als 5 mm unter der Flüssigkeitsoberfläche angeordnet ist, zu fokussieren. Bei Ausführungsbeispielen der Erfindung kann die Detektionsvorrichtung ausgelegt sein, um mehrere Detektionen des jeweiligen Teilvolumens durchzuführen, z.B. mit einer Aufnahmerate zwischen 50 und 150 Hz, beispielsweise 100 Hz. Bei Ausführungsbeispielen kann die Detektionsvorrichtung eine Bildaufnahmevorrichtung mit einer Bildaufnahmerate von 100 Hz sein. Bei Ausführungsbeispielen kann der Fokus der Bildaufnahmevorrichtung verfahren werden, um ein vertikales Scannen in einem begrenzten lateralen Bereich durchzuführen. Bei Ausführungsbeispielen kann eine Verfahrgeschwindigkeit beispielsweise 5 mm/s betragen, während Bilder aufgenommen werden, um Bilder in unterschiedlichen Tiefen aufzunehmen. Bei Ausführungsbeispielen kann das Volumen der Flüssigkeit in dem Fluidbehälter beispielsweise 150 µl betragen, was ein typisches Volumen in einer Zellkultur ist.

Ausführungsbeispiele können auf ein Dispensieren einer dokumentierten Anzahl von Partikeln verwendet werden. Insbesondere können Ausführungsbeispiele der Erfindung bei einer Zelllinienherstellung verwendet werden, um gesichert und dokumentiert einzelne Zellen in Kavitäten einer Mikrotiterplatte abzugeben. Bei Ausführungsbeispielen der Erfindung können einzelne Zellen oder eine bestimmte Anzahl von Zellen zum Nachweis der Monoklonalität und zur Weiterverarbeitung in monoklonale Zellkulturen gedruckt werden. Ausführungsbeispiele können insbesondere zur Einzelzellanalyse verwendet werden.

Ausführungsbeispiele der Erfindung schaffen somit ein Verfahren, bei dem ein Objekt, also eine Zelle oder ein Partikel, in ein definiertes Teilvolumen eines Fluids, mit dem ein Fluidbehälter zumindest teilweise gefüllt ist, abgegeben wird, Schritt 50 in Fig. 4. Das Objekt wird beim Eintritt in das Fluid oder unmittelbar nach dem Eintritt in das Fluid erfasst.

Dazu können ein oder mehrere Detektionen in dem definierten Teilvolumen und/oder in einem oder mehreren Teilvolumen unterhalb des definierten Teilvolumens zeitlich koordiniert mit der Abgabe der zumindest einen Zelle oder des zumindest einen Partikels durchgeführt werden, Schritt 52 in Fig. 4. Die Erfassung des Objekts kann spätestens zehn Sekunden nach dem Eintritt in das Fluid, vorzugsweise spätestens fünf Sekunden und noch vorzugsweiser spätestens eine Sekunde nach dem Eintritt in das Fluid erfolgen. Das definierte Teilvolumen weist bei Ausführungsbeispielen eine Fläche auf, die geringer ist als die Fläche einer Eintrittsöffnung des Fluidbehälters. Bei Ausführungsbeispielen wird das Objekt auf eine Oberfläche des Fluids abgegeben. Bei Ausführungsbeispielen wird das Objekt in ein definiertes Fluidvolumen unterhalb der Oberfläche der Flüssigkeit abgegeben.

Bei Ausführungsbeispielen ist der Dispenser ausgelegt, um Partikel- oder Zellsuspension gezielt an einer vordefinierten Position in den Fluidbehälter zu überführen. Mögliche Mechanismen hierzu sind ein Drop-on-Demand-Drucken (piezo-elektrisch oder thermisch getrieben), eine fluoreszenzbasierte Durchflusssymmetrie (FACS), ein Pipettieren (händisch oder mittels Pipettierroboter), ein Dosierer (ventilbasiert, mittels eines Verdrängers, mittels einer Spritzenpumpe, usw.), oder ein Mikromanipulator. Bei Ausführungsbeispielen ist die Detektionsvorrichtung ausgebildet, um auf den oberen Rand des Flüssigkeitslevels in der Mitte des Fluidbehälters (Reservoirs) zu fokussieren. Damit kann automatisch der Bereich des Auftreffens des Tropfens, d. h. des Transportvolumens mit dem Objekt darin) im Fokus sein. Alternativ kann, wie oben beschrieben wurde, das Flüssigkeitsvolumen im Zentrum des Fluidbehälters von unten beginnend durchfokussiert werden, sobald der Tropfen von dem Dispenser abgegeben wurde. Somit ist gewährleistet, dass sich das Objekt zu einem zufälligen Zeitpunkt im Fokus befindet. Dazu ist es nicht notwendig, den genauen Füllstand des Fluidbehälters zu kennen oder zu detektieren, um den Fokus zu finden.

Um den Meniskus der Flüssigkeit im Reservoir möglichst flach zu halten und somit einen reproduzierbaren Pegelstand der Flüssigkeit in dem Fluidbehälter zu haben, können Reservoire mit speziellen Formen verwendet werden, wie sie beispielsweise in der EP 1 880 764 B1 beschrieben sind. Beispielsweise eignen sich flache Stufen hervorragend, um den Meniskus flach zu ziehen, wenn der Flüssigkeitsbehälter mit einem exakt bekannten Flüssigkeitsvolumen gefüllt ist.

Bei Ausführungsbeispielen der Erfindung kann das Objekt auf die Oberfläche eines Fluids in dem Fluidbehälter aufgebracht oder unterhalb der Oberfläche in das Fluid eingebracht werden. Bei Ausführungsbeispielen erfolgt die Detektion mittels einer Bildaufnahmevorrichtung, die auf ein entsprechendes Teilvolumen fokussiert. Bei alternativen Ausführungsbeispielen können andere Detektionsvorrichtungen verwendet werden. Beispielsweise kann eine Detektionsvorrichtung durch in der Wand des Fluidbehälters gebildete Elektroden implementiert sein, die ausgebildet sind, um eine Kapazität zwischen denselben zu erfassen. Beispielsweise können mehrere solche Elektroden in unterschiedlichen Tiefen des Fluidbehälters angeordnet sein, um eine Detektion in mehreren unterschiedlichen Tiefen durchführen zu können.

Ausführungsbeispiele der Erfindung schaffen somit Vorrichtungen und Verfahren, bei denen ein Observationsvolumen in dem Fluidbehälter (Reservoir) beobachtet wird, das kleiner ist als das Behältervolumen. Bei Ausführungsbeispielen wird nur der Eintrittspunkt des Objekts in das Fluid bzw. die Flüssigkeit observiert. Entsprechend kann ein Sensor zum Beobachten des Observationsvolumens und ein Mechanismus zum gezielten Abgeben von Partikeln ausschließlich in das Observationsvolumen vorgesehen sein. Entsprechend weisen Vorrichtungen und Verfahren zum Erkennen von Partikeln und Zellen in einer Flüssigkeit ein Reservoir bzw. eine Kavität zur Aufnahme von Flüssigkeiten, einen Mechanismus zum Überführen von Flüssigkeiten mit einer oder mehreren Zellen/Partikeln, und einen Sensor zum Erkennen von einzelnen oder mehreren Partikeln oder Zellen auf. Bei Ausführungsbeispielen ist das Reservoir ein Reservoir zur Aufnahme von Partikel- oder Zellsuspensionen, der Mechanismus ist ein Mechanismus zum gezielten Überführen der Partikel- oder Zellsuspension in das Reservoir an einer vordefinierten Position, und der Sensor ist ein Sensor zum Erkennen von einzelnen oder mehreren Partikeln oder Zellen in einem Zellmedium an der vordefinierten Position.

Gemäß Ausführungsbeispielen kann der Sensor ein optischer Sensor oder ein bildgebender Sensor sein. Der bildgebende Sensor kann einen verstellbaren Fokus aufweisen und kann ausgelegt sein, um durch das Observationsvolumen zu fokussieren. Mit anderen Worten kann der bildgebende Sensor ausgelegt sein, um das Observationsvolumen vertikal abzuscannen. Bei Ausführungsbeispielen erfolgt dies von unterhalb des Fluidbehälters, so dass eine Einstellung des Fokus in Tiefenrichtung stattfindet. Bei Ausführungsbeispielen erfolgt das Überführen einer Partikel- oder Zellsuspension in das Fluid in dem Fluidbehälter kontaktfrei in der Form von frei-fliegenden Tropfen. Bei Ausführungsbeispielen weist der frei-fliegende Tropfen ein Volumen von maximal 100 nl auf. Bei Ausführungsbeispielen ist der Fluidbehälter mit einer Flüssigkeit vorbefüllt. Bei Ausführungsbeispielen wird die Flüssigkeit mit der Zelle/dem Partikel in den Fluidbehälter eingebracht. Bei Ausführungsbeispielen ist der Fluidbehälter derart ausgebildet, dass eine darin befindliche Flüssigkeit eine flache Oberfläche (Meniskus) aufweist. Bei Ausführungsbeispielen weist der Fluidbehälter hierzu eine Kante in einer bestimmten Tiefe auf, wobei das Volumen der Flüssigkeit in dem Fluidbehälter derart angepasst ist, dass die Flüssigkeit bis zu der Kante reicht. Bei Ausführungsbeispielen weist der Fluidbehälter ein Volumen von mindestens 100 µl auf.

Bei Ausführungsbeispielen ist ein Mechanismus vorgesehen, durch den der Fluidbehälter, der Dispenser und die Detektionsvorrichtung zueinander verfahren werden können, um nacheinander Zellen/Partikel in mehrere Fluidbehälter einzubringen, beispielsweise in die Wells einer Mikrotiterplatte. Ein solcher Mechanismus kann beispielsweise ausgelegt sein, um die mehreren Fluidbehälter, beispielsweise die Mikrotiterplatte, relativ zu dem Dispenser und der Detektionsvorrichtung zu bewegen, oder um den Dispenser und die Detektionsvorrichtung relativ zu den mehreren Fluidbehältern zu bewegen. Somit ist es möglich, nacheinander Zellen/Partikel in mehrere Fluidbehälter einzubringen und unmittelbar zuverlässig zu erfassen, ob eine Zelle/ein Partikel in jeden der Fluidbehälter gelangt ist.

Die hierin beschriebene Technik bietet gegenüber bekannten Verfahren entscheidende Vorteile.

Ein entscheidender Nachteil bekannter Dispensiertechnologien in Kombination mit Imagern sind die Zeit und die notwendigen Prozessschritte zwischen dem Dispensieren, also dem Zelltransport in den Fluidbehälter (das Well) und dem Abbilden des Well. Hier wurde zunächst eine Einzelzelle in einem Well der Mikrotiterplatte abgelegt, dann wurde die Mikrotiterplatte aus dem Dispenser entnommen, dann wurde die Platte zentrifugiert, dann wurde die Platte in einen Imager eingelegt, und dann erfolgte eine Erfassung durch den Imager. Hinzu kommen noch die schlechten Konditionen bei der Abbildung des Wellbodens aus Gründen der fertigungsbedingten Geometrien der Wells. Diese Nachteile werden durch die hierin beschriebene Technik kompensiert. Die Erfassung erfolgt direkt zusammen mit der Ablage der Zelle, d. h. dem Dispensieren. Die Ablage kann hochpräzise durchgeführt werden, so dass die Zelle an einer vordefinierten Position, beispielsweise zentral, im Well ankommt und direkt am Eintrittspunkt der Flüssigkeit im Well bereits gescannt werden kann. Dadurch muss die Platte nicht bewegt werden, bzw. müssen die Zellen nicht auf den Wellboden zentrifugiert werden. Dadurch können die Schritte des Entnehmens der Platte aus dem Dispenser, des Zentrifugierens der Platte und des Einlegens der Platte in den Imager entfallen. Ferner kann das Erfassen (Scannen) lediglich von unten nach oben unter Verwendung einer Fokusverschiebung am Ort des Eintritts der Zelle in den Fluidbehälter (das Well) erfolgen. Dies kann sehr schnell erfolgen und die Zelle kann immer automatisch im Bild sein.

Ausführungsbeispiele der vorliegenden Erfindung sind daher vorteilhaft dahin gehend, dass das Dispensieren und Detektieren direkt zeitlich nacheinander erfolgen und kein Bewegen der Platte und somit kein unkontrolliertes Bewegen der Zelle im Behälter erfolgt. Zwischen Dispensieren und Detektieren finden keine Zwischenschritte statt. Ferner erfolgt das Erfassen in der freien Flüssigkeit und nicht am Wellboden. Damit gibt es keine Abschattungs- oder Brechungseffekte und keine Geisterbilder von Zellen. Ferner können weitere Nachteile vermieden werden, die eine Erfassung am Wellboden beeinträchtigen können, wie z.B. Kratzer, Fingerabrücke (unten/außen), (elektrostatisch) angezogener Staub (unten/außen) oder Schmutz (Debris) von oben/innen, der beispielsweise mit der Zelle auf den Wellboden zentrifugiert wurde. Bei Ausführungsbeispielen der Erfindung wird ein vertikales statt eines horizontalen Scannens verwendet, so dass keine Fokusprobleme und keine Notwendigkeit, Bilder zusammensetzen, existieren. Das zu scannende Volumen ist wesentlich kleiner als das gesamte Wellvolumen. Im Gegensatz zu einem horizontalen Scannen entfällt ein Verfahren der Platte, was wiederum ein schnelleres Erfassen möglich macht. Schließlich hängt die Bildqualität nicht mehr von der Qualität des Fluidbehälters, z.B. des Mikrotiterplatten-Substrats, bzw. dessen Boden ab, da die Erfassung stattfindet, während sich die Zelle/der Partikel in der freien Flüssigkeit befindet.

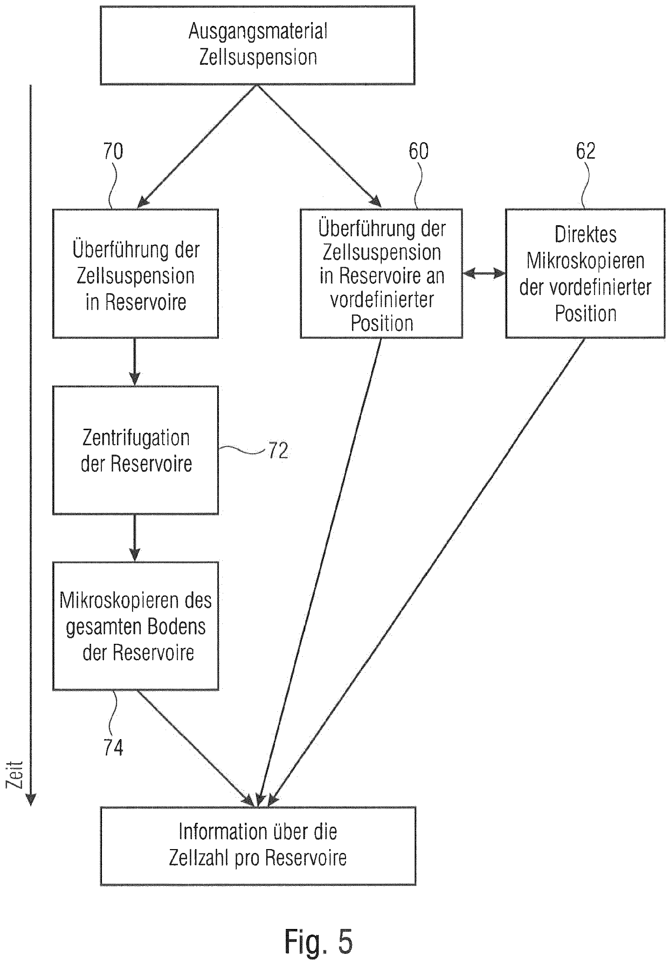

Fig. 5 stellt eine Gegenüberstellung eines hierin beschriebenen Ausführungsbeispiels mit einem bekannten Verfahren dar, wobei von oben nach unten die Zeitachse angetragen ist. Als Ausgangsmaterial dient jeweils eine Zellsuspension. Bei dem hierin beschriebenen Verfahren wird die Zellsuspension an einer vordefinierten Position in Reservoire überführt, Schritt 60. Gleichzeitig erfolgt ein direktes Mikroskopieren der vordefinierten Position, Schritt 62. Als Ergebnis der Schritte 60 und 62 liegen Informationen über die Zellzahl pro Reservoir vor.

Im Gegensatz dazu wird bei bekannten Verfahren die Zellsuspension in Reservoire überführt, Schritt 70, die Reservoire werden zentrifugiert, Schritt 72, und ein Mikroskopieren des gesamten Bodens der Reservoire findet statt, Schritt 74. Somit haben bekannte Verfahren einen deutlich höheren Zeitaufwand, wobei in Fig. 5 die Höhe der einzelnen Prozessblöcke mit der Dauer der entsprechenden Schritte korreliert ist. Es zeigt sich, dass durch die hierin beschriebenen Verfahren eine Vereinfachung und Beschleunigung des Gesamtprozesses erreicht wird.

Ausführungsbeispiele der vorliegenden Erfindung ermöglichen somit bei einer Zellvereinzelung einen verbesserten Nachweis von Einzelzellen, wobei der gesamte Prozess vereinfacht und beschleunigt ist. Gleichzeitig bleibt die Voraussetzung bestehen, dass die Zelle in ein ausreichend großes Volumen abgegeben werden kann, was eine nachfolgende Kultivierung (Wachstum der Zellen) ermöglicht. Ausführungsbeispiele beinhalten einen entsprechenden Schritt eines Zellkultivierung in dem Fluidbehälter nach der Detektion einer Zelle in demselben.

## Patentansprüche

1. Vorrichtung zum Detektieren von Zellen oder Partikeln (16) in einem Fluidbehälter (10), mit folgenden Merkmalen:
einem Dispenser (14), der ausgelegt ist, um zumindest eine Zelle (16) oder zumindest einen Partikel (16) in ein definiertes Teilvolumen (18) eines Fluids (12) abzugeben, mit dem der Fluidbehälter (10) zumindest teilweise gefüllt ist;
einer Detektionsvorrichtung (22, 32), die ausgelegt ist, um zeitlich koordiniert mit der Abgabe der zumindest einen Zelle (16) oder des zumindest einen Partikels (16) durch den Dispenser (14) eine Detektion in dem definierten Teilvolumen (18) und/oder in einem oder mehreren Teilvolumen (18a - 18d) unterhalb des definierten Teilvolumens (18) durchzuführen, um die zumindest eine Zelle (16) oder den zumindest einen Partikel (16) beim Eintritt in das Fluid (12) oder unmittelbar nach dem Eintritt in das Fluid (12) zu erfassen.

2. Vorrichtung nach Anspruch 1, bei der die Detektionsvorrichtung (22, 32) ausgelegt ist, um die Erfassung der zumindest einen Zelle (16) oder des zumindest einen Partikels (16) spätestens 10 Sekunden nach dem Eintritt in das Fluid (12) zu erfassen.

3. Vorrichtung nach Anspruch 1 oder 2, bei der das definierte Teilvolumen (18) eine Fläche (A1) aufweist, die geringer ist als die Fläche (A2) einer Eintrittsöffnung des Fluidbehälters (10).

4. Vorrichtung nach einem der Ansprüche 1 bis 3, bei der der Dispenser (14) ausgelegt ist, um die zumindest eine Zelle (16) oder den zumindest einen Partikel (16) auf eine Oberfläche des Fluids (12) abzugeben.

5. Vorrichtung nach Anspruch 4, bei der die Detektionsvorrichtung (22, 32) ausgelegt ist, um eine Detektion der zumindest einen Zelle (16) oder des zumindest einen Partikels (16) in dem definierten Teilvolumen (18), das eine Tiefe von weniger als 5 mm von der oberen Oberfläche des Fluids (12) aufweist, durchzuführen.

6. Vorrichtung nach einem der Ansprüche 1 bis 4, bei der die Detektionsvorrichtung (22, 32) ausgelegt ist, um beginnend mit einem Teilvolumen (18d) in einer größeren Tiefe nacheinander Detektionen in mehreren Teilvolumen (18 - 18d) mit jeweils abnehmender Tiefe durchzuführen oder um beginnend mit einem Teilvolumen (18) in einer geringeren Tiefe nacheinander Detektionen in mehreren Teilvolumen (18 - 18d) mit jeweils zunehmender Tiefe durchzuführen.

7. Vorrichtung nach einem der Ansprüche 1 bis 6, bei der die Detektionsvorrichtung (22, 32) eine Bildaufnahmevorrichtung (32) aufweist, die ausgelegt ist, um auf das definierte Teilvolumen (18) und/oder das eine oder die mehreren Teilvolumen (18a - 18d) unterhalb des definierten Teilvolumens (18) zu fokussieren und Bilder des definierten Teilvolumens (18) und/oder des einen oder der mehreren Teilvolumen (18a - 18d) unterhalb des definierten Teilvolumens (18) aufzunehmen.

8. Vorrichtung nach Anspruch 7, bei der der Fluidbehälter (10) transparent ist und die Bildaufnahmevorrichtung (22, 32) unterhalb des Fluidbehälters (10) angeordnet ist.

9. Vorrichtung nach einem der Ansprüche 1 bis 8, bei der der Fluidbehälter (10) eine Seitenwand mit einer Kante aufweist, wobei ein Kontaktwinkel des Fluids bezüglich der Seitenwand derart eingestellt ist, dass das Fluid (12) eine flache Oberfläche aufweist.

10. Vorrichtung nach einem der Ansprüche 1 bis 9, die eine Mehrzahl von Fluidbehältern (10) und einen Positionierungsmechanismus aufweist, der ausgelegt ist, um den Dispenser (14), die Detektionsvorrichtung (22, 32) und jeden der Mehrzahl von Fluidbehältern (10) relativ zueinander zu positionieren, um die zumindest eine Zelle (16) oder den zumindest einen Partikel (16) beim Eintritt in das Fluid (12) oder unmittelbar nach dem Eintritt in das Fluid (12) dieses Fluidbehälters (10) zu erfassen.

11. Verfahren zum Detektieren von Zellen oder Partikeln (16) in einem Fluidbehälter (10), mit folgenden Merkmalen:
Abgeben (50, 60) zumindest einer Zelle (16) oder zumindest eines Partikels (16) in ein definiertes Teilvolumen (18) eines Fluids (12), mit dem ein Fluidbehälter (10) zumindest teilweise gefüllt ist;
Durchführen (52, 62) einer Detektion in dem definierten Teilvolumen (18) und/oder in einem oder mehreren Teilvolumen (18a - 18d) unterhalb des definierten Teilvolumens (18) zeitlich koordiniert mit der Abgabe der zumindest einen Zelle (16) oder des zumindest einen Partikels (16), um die zumindest eine Zelle (16) oder den zumindest einen Partikel (16) beim Eintritt in das Fluid (12) oder unmittelbar nach dem Eintritt in das Fluid (12) zu erfassen.

12. Verfahren nach Anspruch 11, bei dem die Erfassung der zumindest einen Zelle (16) oder des zumindest einen Partikels spätestens 10 Sekunden nach dem Eintritt in das Fluid (12) erfolgt.

13. Verfahren nach Anspruch 11 oder 12, bei dem das definierte Teilvolumen (18) eine Fläche (A1) aufweist, die geringer ist als die Fläche (A2) einer Eintrittsöffnung des Fluidbehälters (10).

14. Verfahren nach einem der Ansprüche 11 bis 13, bei dem die zumindest eine Zelle (16) oder der zumindest eine Partikel (16) auf eine Oberfläche des Fluids (12) abgegeben wird.

15. Verfahren nach Anspruch 14, bei dem eine Detektion der zumindest einen Zelle (16) oder des zumindest einen Partikels (16) in dem definierten Teilvolumen (18), das eine Tiefe von weniger als 5 mm von der oberen Oberfläche des Fluids (12) aufweist, durchgeführt wird.

16. Verfahren nach einem der Ansprüche 11 bis 14, bei dem beginnend mit einem Teilvolumen (18d) in einer größeren Tiefe nacheinander Detektionen in mehreren Teilvolumen (18 - 18c) mit jeweils abnehmender Tiefe durchgeführt werden oder beginnend mit einem Teilvolumen in einer geringeren Tiefe (18) nacheinander Detektionen in mehreren Teilvolumen (18a - 18d) mit jeweils zunehmender Tiefe durchgeführt werden.

17. Verfahren nach einem der Ansprüche 11 bis 16, bei dem das Durchführen einer Detektion ein Fokussieren auf das definierte Teilvolumen (18) und/oder das eine oder die mehreren Teilvolumen (18a - 18d) unterhalb des definierten Teilvolumens (18) und ein Aufnehmen von Bildern des definierten Teilvolumens (18) und/oder des einen oder der mehreren Teilvolumen (18a - 18d) unterhalb des definierten Teilvolumens (18) aufweist.

18. Verfahren nach Anspruch 17, bei dem der Fluidbehälter (10) transparent ist und das Durchführen einer Detektion mittels einer Bildaufnahmevorrichtung (32) erfolgt, die unterhalb des Fluidbehälters (10) angeordnet ist.

19. Verfahren nach einem der Ansprüche 11 bis 18, das ein Positionieren eines Dispensers (14), einer Detektionsvorrichtung (12) und jedes mehrerer Fluidbehälter relativ zueinander aufweist, um die zumindest eine Zelle oder den zumindest einen Partikel beim Eintritt in das Fluid oder unmittelbar nach dem Eintritt in das Fluid dieses Fluidbehälters zu erfassen.
